# EUROPEAN PATENT SPECIFICATION

(11) **EP 1 198 476 B1**
(45) Date of publication and mention of the grant of the patent: **06.09.2006**
(21) Application number: 00942486.2
(22) Date of filing: 11.07.2000
(51) Int. Cl.: C07K 14/47, C12N 15/62, C12Q 1/68

(54) **DEATH EFFECTOR DOMAIN OF MAMMALIAN APOPTOSIS MEDIATOR, FADD, INDUCING BACTERIAL CELL DEATH**
"DEATH-EFFECTOR"-DOMÄNE DES SÄUGETIER-APOPTOSE-MEDIATORS, FADD, DIE BAKTERIELLEN ZELLTOD AUSLÖST
DOMAINE EFFECTEUR DE MORT DU MEDIATEUR DE L'APOPTOSE MAMMIFERE, FADD, PROVOQUANT LA MORT DES CELLULES BACTERIENNES

(30) Priority: 12.07.1999 KR 9927964
(43) Date of publication of application: 24.04.2002
(73) Proprietor: Imagene Co., Ltd., Seocho-ku, Seoul 137-070 (KR); Korea Institute of Science and Technology, Seoul 136-791 (KR)
(72) Inventor: KIM, Sunghoon, Jangangu, Suwon, Kyunggido 440-746 (KR); KIM, Key-Sun, Seoul 130-650 (KR)
(74) Representative: Prato, Roberto
(86) International application number: PCT/KR2000/000721
(87) International publication number: WO 2001/004153

(56) References cited:
- WO-A1-99/00499
- CHEMICAL ABSTRACTS, vol. 125, no. 1, 01 July 1996, Columbus, Ohio, US; abstract no. 8362Q, ZHANG ET AL.: 'A mouse Fas-associated protein with homology to the human Mort 1/FADD protein is essential for Fas-induced apoptosis' page 965; column LEFT; XP002953080 -& MOL. CELL. BIOL. vol. 16, no. 6, 1996, (ENG), pages 2756 - 2763, XP010320434
- CHEMICAL ABSTRACTS, vol. 129, no. 19, 09 November 1998, Columbus, Ohio, US; abstract no. 243075Q, CHOI K.B. ET AL.: 'Lipopolysaccharide mediates endothelial apoptosis by a FADD-dependent pathway' page 459; column LEFT; XP002953081 & J. BIOL. CHEM. vol. 273, no. 32, 1998, (ENG), pages 20185 - 20188
- LEE SANG-WON ET AL: 'Death effector domain of a mammalian apoptosis mediator, FADD, induces bacterial cell death' MOLECULAR MICROBIOLOGY vol. 35, no. 6, March 2000, pages 1540 - 1549

## Description

### TECHNICAL FIELD

The present invention relates to a death effector domain of mammalian apoptosis mediator, FADD-DED of seq. ID NO:3 inducing bacterial cell death.

### BACKGROUND ART

Apoptosis is considered as an active self-destructive process working in multicellulat higher eukaryotes (Steller, 1995; Ameisen, 1996). However, this process has been also reported in unicellular eukaryotic organisms, such as, *Trypanosome* (protozoa), slime mold *Distyostelium* and free-living ciliate *Tetrahymena* (Wheatley *et al*., 1993; Cornillon *et al*., 1994; Welbum *et al*., 1999) and even in prokaryotic organism (Naito *et al*., 1995; Hochman, 1997).

Conservation of apoptosis throughout different phylogenetic lines implies that this process may have evolved from a common origin. Bax is a mammalian pro-apoptotic protein. Expression of this factor generates reactive oxygen species (ROS) and decreases mitochondrial membrane potential (Xiang *et al*., 1996).

A trace amount of human Bax in *E. coli* induced cell death with physiological changes similar to those in human cell (Asoh *et al*., 1998; Ishibashi *et al*., 1998). These results implied that at least some mammalian molecules involved in apoptosis may affect the viability of bacterial cells in a manner similar to their functions in mammalian cells.

In the present invention, we employed a well-known key mediator of mammalian apoptosis, FADD, to investigate whether this molecule causes bacterial cell death and if so, what is the cause for the death. FADD consists of two functional domains that have been determined by deletion mapping (Chinnaiyan *et al*., 1995). Its C-terminal domain is called death domain (DD) and recruited to DD of FAS that is oligomerized by Fas ligand (Chinnaiyan *et al*., 1996). The N-terminal death effector domain (DED) of FADD is then associated with its homologous domain of FLICE (procaspase-8), triggering the downstream pro-apoptotic cascade (Cohen, 1997; Medema *et al*., 1997; Juo *et al*., 1998).

DED of FADD contains six anti-parallel, amphipathic α-helices (Eberstadt *et al*., 1998) and shows an overall structural similarity to DDs of FADD (Jeong *et al*., 1999) and FAS (Huang *et al*., 1996). Overexpression of FADD-DED alone induces mammalian cell death independent of an apoptotic signal through Fas (Muzio *et al*., 1996; Medema *et al*., 1997).

The inventors consider that mammalian pro-apoptotic molecules can trigger a bacterial cell death that is similar to mammalian cell death. If so, bacterial cell may be a useful system to investigate the mammalian apoptosis and to screen the molecules related to apoptosis.

As the first step to explore this possibility, the inventors determine the structural domains of FADD by proteolytic mapping and test whether these domains can trigger bacterial cell death. The results suggest that the death effector domain of mammalian pro-apoptotic molecule, FADD, induces bacterial cell death by enhancing the cellular level of ROS.

### DISCLOSURE OF INVENTION

The object of the present invention is to provide a death effector domain, FADD-DED consisting of SEQ ID NO : 3 of mammalian apoptosis mediator, wherein FADD-DED induces bacterial cell death.

Also, the invention provides a kit for screening the molecules related to apoptosis comprising an expression vector comprising a polynucleotide encoding exclusively the FADD-DED consisting of SEQ ID NO:3 of mammalian apoptosis mediator which induces bacterial cell death, and T7 promoter.

FADD is a mammalian pro-apoptotic mediator consisting of the N-terminal death effector domain (DED)(SEQ ID NO : 3) and the C-terminal death domain (DD)(SEQ ID NO : 4). The N-terminal 88 residue fragment of murine FADD is defined as the stable structural unit of DED as determined by proteolytic digestion and conformational analysis.

This domain induces bacterial as well as mammalian cell death whereas the full-length or DD of FADD does not. The *Escherichia coli* cells expressing FADD-DED show elongated cell morphology and increased level of nicked chromosomal DNA and mutation. The lethality of FADD-DED is abolished by co-expression of thioredoxin and superoxide dismutase or relieved by the addition of vitamin E as reducing agents and in anaerobic growth condition.

The toxicity of FADD-DED is genetically suppressed by various oxidoreductases of *E. coli*. All of these results suggest that death effector domain of mammalian FADD induced bacterial cell death by enhancing cellular level of Reactive Oxygen Species (ROS).

Although DED and DD are located in the N- and C-terminal region of FADD, their exact structural boundaries have not been well defined. In the present invention, we employ proteolytic cleavage to define the structural units for DED and DD of FADD.

The subtilisin cleavage of FADD generated two proteolysis-resistant peptide fragments. One of the resulting peptides from Ala⁸⁹ to Ser¹⁸³ formed a stable folding unit of DD whose structure was recently solved (Jeong *et al*., 1999).

This result suggests that the other domain spanning from Met¹ to Thr⁸⁸ should be a structural unit for DED. Although this domain is a little larger than the size of DED previously determined by deletion mapping (Chinnaiyan *et al*., 1996), it forms a stable α-helical conformation as determined by CD analysis.

Point mutations of FADD-DED abolish its lethal effect, implying that the structural integrity of FADD-DED is important for the lethal activity. Human FADD-DED consists of six antiparallel helices.

The substitution mutation at Phe²⁵ located in helix α2 abolishes its lethal activity both in *E*. *coli* and mammalian cells (Eberstadt *et al*., 1998). It suggests that the same protein surface of FADD-DED may be used in triggering the lethal activity. Other two mutations at Asp¹⁹ and Asp⁷⁴ are also expected to affect on the interaction surface that is not yet known clearly. Base on these results, it appears that the lethality of FADD-DED is due to its intrinsic biological activity.

Expression of FADD-DED induces cell elongation and DNA damage, implying that the enhanced level of ROS is responsible for these results. To confirm this possibility, we employ reducing proteins and reagent or anaerobic condition to test whether these treatments can neutralize the lethal effect of FADD-DED. ROS can be removed by the thioredoxin/thioredoxin reductase system (Fernando *et al*., 1992; Das *et al*., 1997).

Thioredoxin is a detoxification agent against oxidative stress and involved in oxidative cell death pathway in mammalian cells (Spector *et al*., 1988; Fernando *et al*., 1992). It inhibits the oxidative cell death by binding to ASK-1 (apoptosis singal-regulating kinase 1), a kinase leading to cell death. Thioredoxin is reduced by a thioredoxin reductase coupled to NADPH in normal cell (Lennon and Williams, 1996; Prinz *et al*., 1997; Takemoto *et al*., 1998). Likewise, SOD protects oxidative DNA damage (Keyer *et al*., 1995).

Vitamin E (Fuentes and Amabile-Cuevas, 1998) or anaerobic condition partially also relieves the killing effect of FADD-DED. In addition, most of the *E. coli* genes that suppressed the lethality of FADD-DED are related to the redox potential of the cell. All of these results strongly suggest the involvement of ROS in cell death triggered by FADD-DED.

Alternatively, FADD-DED may induce the bacterial cell death via deadly interactions with essential *E. coli* proteins. However, this possibility is not supported for the following reasons.

First, if the lethal effect of FADD-DED are blocked by physical interactions with *E*. *coli* proteins, these proteins would not be metabolically related. However, the selected suppressors are mostly clustered to oxidation/reduction processes.

Second, we employ yeast two hybrid system (Gyuris *et al*., 1993) to screen the *E*. *coli* proteins that may interact with FADD-DED. However, no proteins are selected to interact with FADD-DED even after screening of more than 20,000 clones.

Third, since eukaryotic DED can make homotypic interactions with the homologous domains (unpublished data) (Cohen, 1997; Medema *et al*., 1997; Juo *et al*., 1998), FADD-DED may bind to the *E. coli* proteins containing its homologous domain. However, *E*. *coli* proteins with the homologous domain have not been identified so far (Aravind *et al*., 1999).

Based on these observations, a fortuitous lethal interaction between FADD-DED and *E*. *coli* protein(s) is unlikely.

Combined all together, it can be concluded that FADD-DED would induce bacterial cell death by enhancing the level of ROS with yet unknown mechanism. Since ROS works both in prokaryotic and eukaryotic systems as a death mediator, *E*. *coli* may be used as a convenient system to investigate the molecular mechanism of cell death.

### BRIEF DESCRIPTION OF DRAWINGS

FIG 1 shows proteolytic digestion of the purified recombinant murine FADD and conformational analysis of FADD-DED.
FIG 2 shows death effector domain of FADD which induces bacterial and mammalian cell death.
FIG 3 shows the *E*. *coli* cell death which depends on expression of FADD-DED.
FIG 4 shows morphological change of *E*. *coli* induced by FADD-DED.
FIG 5 shows chromosomal damage and mutation frequency increased by expression of FADD-DED in *E. coli*.
FIG 6 shows the effect of thioredoxin and SOD on the cell growth of *E. coli* expressing FADD-DED.
FIG 7 shows the effect of vitamin E on the lethal effect of FADD-DED.

### BEST MODE FOR CARRYING OUT THE INVENTION

### 1. Proteolytic and conformatinal analyses of death effector domain of FADD

Deletion analysis suggests that DED and DD of FADD span from Phe⁴ to Leu⁷⁹ and from Val¹⁰⁴ to Val¹⁷⁷, respectively (Chinnaiyan *et al*., 1995). However, it is not known whether these domains are equivalent to their exact structural units. Here, we subject murine FADD to subtilisin digestion to define the structural domains of FADD. The peptides forming a compact conformation would be resistant to the proteolysis of subtilisin.

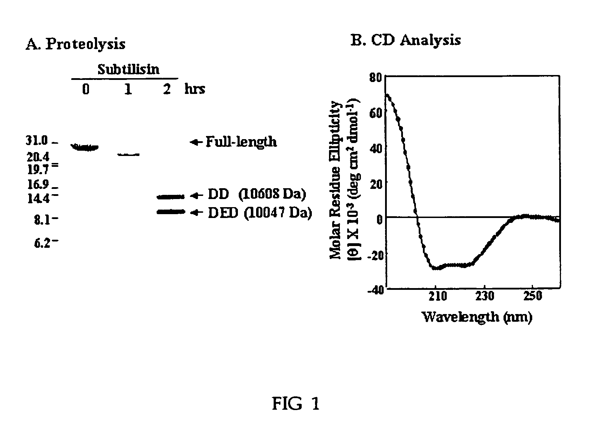

The subtilisin-digested peptide fragments of FADD are separated by electrophoresis to determine their N-terminal amino acid sequences and molecular weights. The two peptide fragments remain intact after 2 hours digestion, suggesting that these peptides would form stable folding units resistant to proteolysis (FIG 1A).

As shown in FIG 1A, the purified murine FADD is digested with subtilisin and the digested protein is sampled in 1 and 2 hours. The resulting peptides are resolved by SDS-polyacrylamide gel electrophoresis. The two peptides resistant to subtilisin digestion are generated after 2 hours digestion.

The N-terminal amino acid sequences and molecular weights of the peptides are determined by Edman degradation and mass spectroscopy, respectively. The upper and lower bands in the gel are determined to be death effector domain (DED) from Met¹ to Thr⁸⁸ and death domain (DD) from Ala⁸⁹ to Ser¹⁸³, respectively.

These peptides are isolated by HPLC and their N-terminal amino acid sequences and molecular weights are determined. The results indicate that the upper and lower bands in the gel are the peptides spanning the C-terminal 95 residues (residues Ala⁸⁹-Ser¹⁸³) and the N-terminal 88 residues (residues Met¹-Thr⁸⁸), respectively.

We already showed that the C-terminal peptide forms a stable structure of DD (Jeong *et al*., 1999) and thus thought that the subtilisin digestion well defined the structural domains of FADD. We then investigate the conformation of the isolated FADD-DED by circular dichroism.

The CD spectrum obtained from 190 to 260 nm is indicative of α -helical structure (FIG 1B), consistent with its three dimensional structure determined by NMR (Eberstadt *et al*., 1998). The melting temperature of this domain is determined to be approximately 60°C. Based on the results of proteolytic and conformational analyses, FADD-DED appears to form a stably folded structure.

As shown in FIG 1B, the conformation of the isolated FADD-DED is determined by circular dichroism. The CD spectrum is shown as molar residue ellipticity versus wavelength.

### 2. N-terminal death effector domain of FADD induces bacterial cell death

Overexpression of the full length or DED of FADD induces mammalian cell death while its DD does not (Chinnaiyan *et al*., 1995). Here, we test whether the full-length and proteolytically determined DED and DD of murine FADD affect the viability of *E. coli.*

The cDNAs encoding the full-length, DED and DD of FADD are cloned to the *E*. *coli* expression vector containing T7 promoter. All of these constructs do not affect the cell viability when the plasmids are maintained in *E. coli* DH5a in which T7 polymerase is not present.

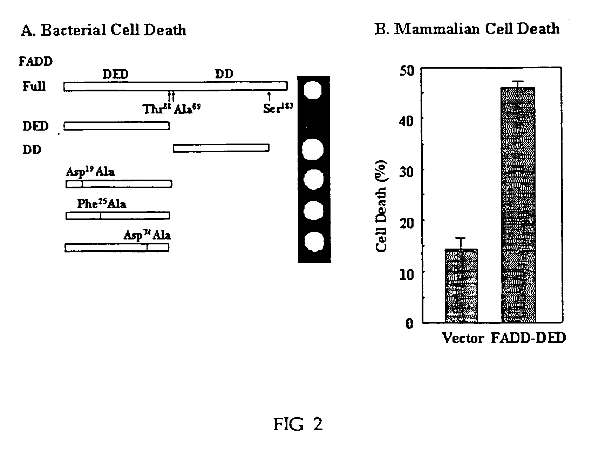

The inventors then introduce these constructs into *E. coli* BL21(DE3) in which T7 polymerase is expressed by IPTG induction. The transformants of *E. coli* BL21(DE3) containing pET3d-FADD-DED are not obtained, whereas the same strain cells containing pET3d-FADD or pET14b-FADD-DD are generated (FIG 2A).

As shown in FIG 2A, the plasmids expressing the full-length, DED and DD of FADD are transformed into *E*. *coli* BL21(DE3). Their effect on the cell viability is determined by colony formation on LB plate containing ampicillin. Three substitution mutants are generated and also tested for their lethality.

Although *E. coli* BL21(DE3) is not supposed to express T7 polymerase unless ITPG is added, it may contain a low level of T7 polymerase resulted from a leaky expression. We consider that the cells containing pET3d-FADD-DED are killed by a trace amount of FADD-DED that is expressed by low level of T7 polymerase.

To determine whether the toxicity of FADD-DED is specific to its structural integrity, we introduce substitution mutations to FADD-DED and test their effect on the lethality of FADD-DED. Phe²⁵ and Asp⁷⁴ are located in helices 2 and 6, respectively and Asp¹⁹ is located between helices 1 and 2.

All of these residues are located on the surface of the protein and conserved among DEDs and DDs of various death molecules, implicating their functional importance (Eberstadt *et al*., 1998). None of these substitution mutants induces cell death (FIG 2A) although they are stably expressed as determined by immunoblotting. Further deletion of FADD-DED or tagging of heterologous peptide to this domain also abolished the lethal activity.

Its extreme toxicity at trace amount and the loss of its toxicity by various mutations strongly suggest that the lethal effect of FADD-DED requires its native conformation and is related to its intrinsic biological function, but is not due to its misfolding.

We then test whether FADD-DED can also induce mammalian cell death. The HeLa cells transfect with pcDNA3-FADD-DED and the vector alone shows about 45% and 15% cell death, respectively (FIG 2B). Thus, FADD-DED consisting of the N-terminal 88 amino acids induces the bacterial as well as mammalian cell death.

As shown in FIG 2B, mammalian vector pcDNA3 containing the gene encoding FADD-DED is transfected into HeLa cells and the apoptotic cells are counted as described in experimental procedures.

### 3. Expression of FADD-DED induced cell morphology change

Since we consider that the lethal effect of FADD-DED is due to its leaky expression, the structural gene of FADD-DED is transferred into another expression vector, pET28a that contains *lacI* gene to control the expression of T7 polymerase more tightly. As expected, the *E*. *coli* BL21(DE3) containing pET28a-FADD-DED is viable on LB plate in the absence of IPTG but not in the presence of IPTG. *E*. *coli* BL21(DE3) containing pET28a-FADD-DED is grown in LB broth without IPTG.

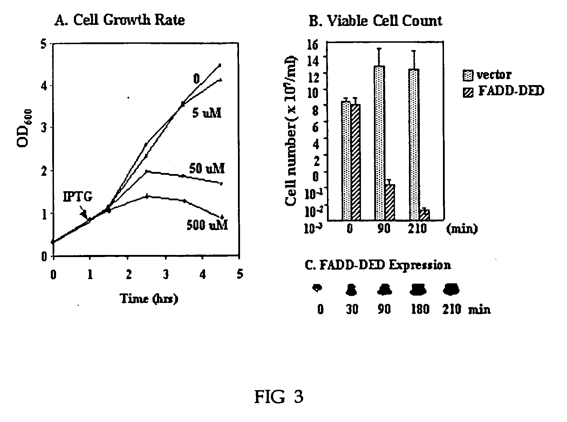

The expression of FADD-DED is then induced with different concentrations of IPTG. The cell growth is more severely inhibited according to the amount of the added IPTG (FIG 3A). We then add 500 µM IPTG to the *E*. *coli* cells containing pET28a and pET28a-FADD-DED and compare the number of viable cells at time interval.

As shown in FIG 3A, *E*. *coli* BL21(DE3) containing pET28a-FADD-DED is grown in LB broth and expression of FADD-DED is induced by adding the indicated amounts of IPTG. The cell growth is monitored by OD₆₀₀.

Almost all of the *E*. *coli* cells containing pET28a-FADD-DED lose the viability after IPTG induction while those containing pET28a alone are still viable, although the two *E. coli* stains are both viable at the same level at the time of IPTG induction (FIG 3B).

As shown in FIG 3B, *E. coli* BL21(DE3) containing pET28a-FADD-DED is grown in LB broth in the presence of 0.5 mM IPTG and the cells are sampled at time interval and plated on LB to count the viable cell.

The cellular level of FADD-DED is increased according to the time course after IPTG induction (FIG 3C). These results indicate that the expression of FADD-DED is responsible for the cell death of *E. coli*.

As shown in FIG 3C, expression of FADD-DED is induced from *E*. *coli* BL21(DE3) containing pET28a-FADD-DED and determined by immunoblotting with anti-FADD-DED antibody.

We then investigate whether the morphology of the *E*. *coli* cells is changed by the FADD-DED expression. Expression of Bax generates reactive oxygen species (ROS) and decreases mitochondrial membrane potential in mammalian cells (Xiang *et al*., 1996), and also gives a similar effect in *E. coli* cells *(Asoh et al*., 1998).

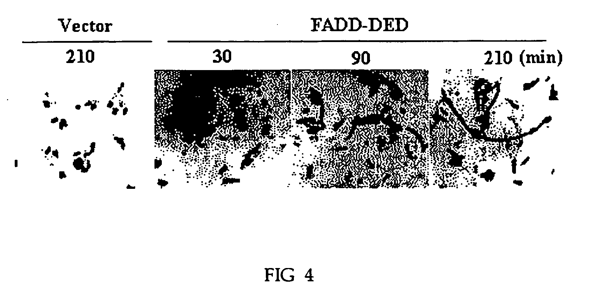

The production of ROS causes elongation of the *E*. *coli* cells (Brandi^{a} *et al*., 1989). If FADD-DED induces ROS production in *E*. *coli*, we consider that it might cause the similar effect on the morphology of *E*. *coli* as Bax. The cells are gradually elongated and the frequency of the elongated cells is increased according to the time course after IPTG induction (FIG 4). In contrast, the *E. coli* cells containing the vector alone show the normal morphology.

As shown in FIG 4, expression of FADD-DED is induced with 0.5 mM IPTG and the morphological change is monitored by microscopy.

Although the similar morphological change by other toxic agents has been also reported (Phillips *et al*., 1967; Suzuki *et al*., 1967; Hrebenda *et al*., 1985; Brandi^{b} *et al*., 1989), we consider that the cell elongation have resulted from the increased ROS based on the functional analogy of FADD and Bax in apoptosis.

### 4. DNA damage and mutation frequency are increased by expression of FADD-DED

The enhanced ROS may also increase chromosomal damage and mutation (Moody and Hassan, 1982; Ames and Gold, 1991). The chromosomal damage in *E*. *coli* cells containing pET28a-FADD-DED and the vector alone is determined by measuring the nicked DNAs.

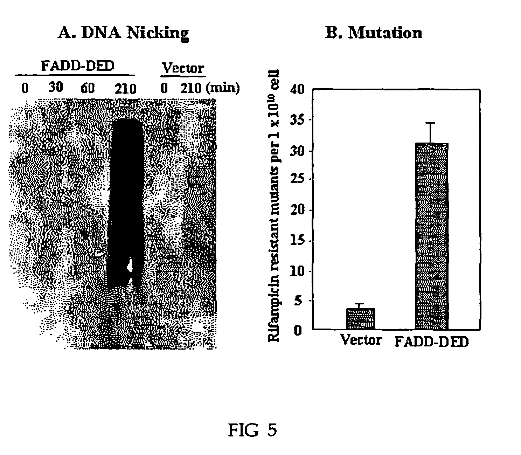

The chromosome is alkaline denatured and the nicked DNAs are isolated and resolved by gel electrophoresis. The amount of the nicked DNA is determined by hybridization using random *E*. *coli* genomic fragments as a probe. The nicked DNA becomes apparent 210 min after induction of FADD-DED whereas almost no nicked DNA is detected in the cells containing the plasmid alone (FIG 5A).

As shown in FIG 5A, the expression of FADD-DED is induced by 0.5 mM IPTG in *E. coli* BL21(DE3) containing pET28a-FADD-DED or the vector alone. The nicked chromosomal DNAs are separated from the cells at indicated times after IPTG induction and detected by Southern blotting with random *E. coli* genomic fragments as described in experimental procedures.

The DNA damage induced by FADD-DED is also determined by comparing the mutation frequency between the *E. coli* cells containing pET28a-FADD-DED and pET28a alone. After incubating these cells on LB plate, they are pooled and re-spread on LB plates with and without rifampicin that is chosen as a marker of mutation (Asoh *et al*., 1998). The rifampicin-resistant cells are generated at approximately 3.2 x 10⁻⁹ and 4 x 10⁻¹⁰ from the cells containing pET28a-FADD-DED and pET28a, respectively (FIG 5B).

As shown in FIG 5B, the *E*. *coli* BL21(DE3) cells containing pET28a-FADD-DED are incubated on LB plate until they form colonies. They are then pooled and spread on LB plates with and without rifampicin (The rifampicin resistance is used as a marker for mutaion). The rifampicin-resistant colonies are counted and divided by the number of the colonies formed on LB plate without rifampicin.

This result suggests that the occurrence of rifampicin-resistant cells are increased about 10 folds even by a trace level expression of FADD-DED.

### 5. The lethal effect of FADD-DED is abolished by reducing agents

We then test whether the lethality of FADD-DED is abolished by proteins that can quench ROS. Thioredoxin is a protein that can control cellular redox potential by the formation of disulfide bond between two cysteines located in the active site. We introduce pET28a-FADD-DED into *E*. *coli* BL21(DE3) overexpressing thioredoxin and the lethal effect of FADD-DED is tested by IPTG induction.

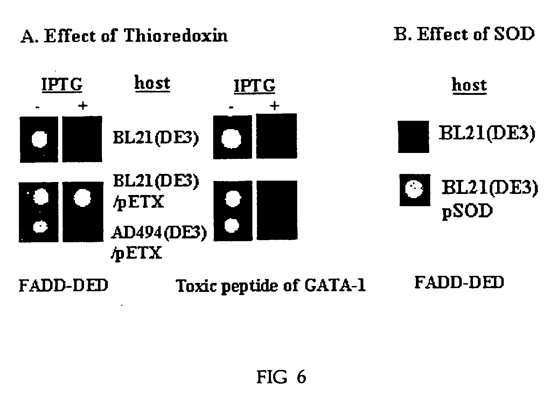

The *E. coli* cells overexpressing thioredoxin grow well on the ITPG-containing LB plate, while the same strain without thioredoxin does not (FIG 6A).

As shown in FIG 6A, the chloramphenicol-resistant plasmid pETX expressing thioredoxin is transformed into *E*. *coli* BL21(DE3) and AD494(DE3) (Novagen) that is thioredoxin reductase defective mutant of BL21(DE3). The plasmid pET28a-FADD-DED is then transformed into each of these strains and the transformants are incubated on LB plate containing the selective antibiotics in the absence and presence of IPTG. The plasmid expressing a toxic peptide derived from mouse transcription factor GATA-1 is also transformed into the same tester strains and its expression is also induced by IPTG.

This result suggests that the lethal effect of FADD-DED is abolished by high level of thioredoxin.

To see whether the reduced form of thioredoxin is important for this quenching effect, pET28a-FADD-DED is transformed into the *E. coli* AD494(DE3) strain in which thioredoxin reductase is defective. Since this strain lacks thioredoxin reductase, the reduced form of thioredoxin is not made. As expected, this cell does not form a colony on the IPTG-containing LB plate, indicating that the reducing potential of thioredoxin is essential for the suppression of the FADD-DED lethal activity.

We also test the lethal effect of the domain derived from mouse transcription factor GATA-1 to see whether this peptide also induces ROS (Trudel *et al*., 1996). The peptide consisting of a zinc finger and basic amino acids binds to the bacterial replication origin to give toxic effect.

The *E. coli* cells expressing this peptide do not form a colony on the IPTG-containing plate as previously reported. However, the lethal effect of this peptide is not neutralized by co-expression of thioredoxin (FIG 6A).

This result means that the induction of ROS is specific to FADD-DED. To further confirm the involvement of the FADD-DED induced ROS in cell killing, we also express FADD-DED in E. *coli* BL21(DE3) expressing superoxide dismutase (SOD) of Aquifex pyrophilus (Lim *et al*., 1997). Expression of this enzyme also quenches the killing effect of FADD-DED (FIG 6B).

As shown in FIG 6B, the effect of SOD on the lethality of FADD-DED is tested. The FADD-DED gene is cloned in pACYC184 and transformed into *E. coli* BL21(DE3) with and without pSOD (the plasmid expressing SOD of *Aquifex pyrophilus)* and the transformants are detected on each of the selected plates.

To further confirm the involvement of the FADD-DED induced ROS in cell killing, we also express FADD-DED in E. *coli* BL21 (DE3) overexpressing superoxide dismutase (SOD). Overexpression of this enzyme also quenches the killing effect of FADD-DED (FIG 6B).

### 6. Lethality of FADD-DED is relieved by depletion of oxygen

If ROS increased by FADD-DED is a real cause for the cell death, the lethal effect of FADD-DED would be relieved by scavenging ROS or by the depletion of oxygen.

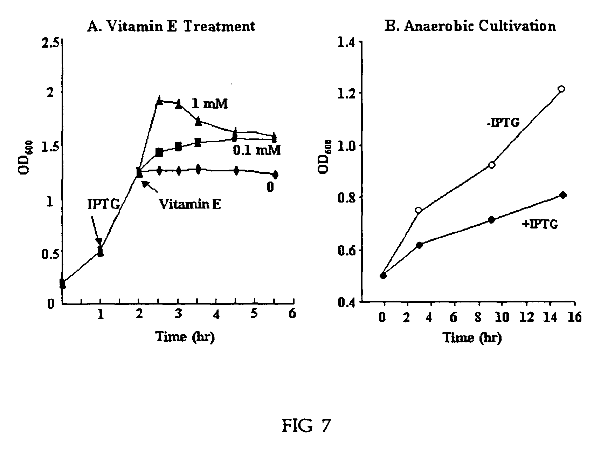

To explore this possibility, we first cultivate *E*. *coli* BL21(DE3) expressing FADD-DED in the presence of reducing agent, vitamin E. We then add vitamin E as a reducing agent after inducing FADD-DED with IPTG in *E. coli* BL21(DE3) to see whether this compound can suppress the toxic effect of FADD-DED. While the cells stop the growth 1 hour after IPTG induction in the absence of vitamin E, the growth is continued in the presence of vitamin E (FIG 7A).

As shown in FIG 7A, the *E*. *coli* BL21(DE3) containing pET28a-FADD-DED is cultivated in LB broth containing ampicillin. Expression of FADD-DED is induced by the addition of 0.1 mM IPTG and then vitamin E (0.1 and 1 mM) is added to the growth media after 1 hour. The cell growth is monitored by OD₆₀₀.

The same cells are cultivated in a partially anaerobic condition. The cells continue to grow after the induction of FADD-DED although the growth is retarded (FIG 7B).

As shown in FIG 7B, the same cells are cultivated in air sealed tubes without agitation until OD₆₀₀ reached 0.5. The cell growth is then monitored at time interval after the addition of IPTG at 0.5 mM.

All of these results support that ROS induced by FADD-DED is responsible for cell death.

### 7. Genetic suppression of the lethal effect of FADD-DED

ROS appears to be a common mediator for both of mammalian and bacterial cell death (Hochman, 1997; Storz and Imaly, 1999). Fas activation increases ROS via NADPH oxidase (Suzuki *et al*., 1998). FADD is the immediate downstream effector of Fas and interacts with the homologous domain of FLICE (pro-caspase-8) (Cohen, 1997; Medema *et al*., 1997; Juo *et al*., 1998).

The activated caspase-8 then cleaves BID that is then translocated into mitochondria, damaging its membrane potential (Li *et al*., 1998). These reports support the notion that ROS may be involved in FADD-mediated mammalian cell death.

The results of the experiments we carried out suggest FADD-induced ROS is responsible for bacterial cell death as well. If this is the case, the *E*. *coli* proteins that can control the cellular level of ROS may suppress the lethal effect of FADD-DED.

To explore this possibility, we co-expressed the *E*. *coli* genomic library with FADD-DED to select the genes that can restore the cell viability. The majority of the selected genes encode the proteins involved in the metabolism of oxidation and reduction such as cytochrome O oxidase, aldehyde dehydrogenase A/glyceraldehydes 3-phosphate dehydrogenase, NADH dehydrogenase I chain and an open reading frame encoding a protein homologous to 2-keto-3-deoxygluconate oxidoreductase of *Bacillus subtilis* (Table 1).

**Table 1. The E. coli genes selected to suppress the lethality of FADD-DED**

| GENES | FUNCTIONS | FREQ. |
|---|---|---|
| *cyoB* | cytochrome O ubiquinol oxidase subunit I | 3 |
| *aldA*/*gapC* | Aldehydedehydrogenase glyceraldehydes 3-phosphate dehydrogenase | 2 |
| *nuoK*/*J*/*I*/*H* | NADH dehydrogenase I chain K/J/I/H | 1 |
| ORF (62 min) | homologue to 2-keto-3-deoxygluconate oxidoreductase of *B. subtilis* | 2 |
| *yeeS* | *radC* family | 1 |

As shown in Table 1, the *E*. *coli* genes are co-expressed with FADD-DED to screen those that can suppress the lethality of FADD-DED. The plasmids containing the *E. coli* genes are isolated from the viable cells and the selected *E. coli* genes are identified. FREQ. stands for the number of identical clones selected.

These oxidoreductases are expected to modulate the cellular redox potential that can buffer the toxic effect of ROS induced by FADD-DED. *A radC* family protein (*yeeS*) is also selected. Although the function of this protein is not completely clear, it may be involved in repairing DNA damaged by ROS (Felzenszwalb *et al*., 1987).

In addition, a few genes encoding proteins of unknown functions are also selected. Although the detailed mechanisms for the suppression of these genes on the lethal effect of FADD-DED need further investigation, the selection of these suppressors are consistent with the involvement of ROS in bacterial cell death.

### EXAMPLES

### (Example 1) Proteolysis and Circular Dichroism Analysis of FADD-DED

The recombinant murine FADD was induced in *E. coli* BL21(DE3) with 0.4 mM IPTG at 37°C for 3 hours from pET3d (Novagen) containing its structural gene. The cells were harvested and disrupted by ultrasonication in a 50 mM Tris-HCl (pH 8.0), 1 mM EDTA, 1 mM DTT, 10% glycerol, and 1 mM phenylmethylsulfonyl fluoride. The proteins were precipitated by adding ammonium sulfate up to 30% to the soluble fraction of the cell extract. The precipitant was collected by centrifugation and dialyzed overnight at pH 4.0 and further purified by reverse phase high performance liquid chromatography (HPLC) using C₈ Vydac column. The purified FADD protein was digested with subtilisin for 2 hours at 15°C in phosphate buffer at pH 8.0. The digested peptides were resolved in 15% SDS-polyacrylamide gel and transferred onto PVDF membrane (MSI). The N-terminal amino acid sequence and molecular weight of the peptides were determined by Procise491 amino acid sequencer (ABI) and matrix-assisted laser desorption/ionization-time of flight (MALDI-TOF) mass spectroscopy (Hewlett-Packard, HPG2025A), respectively. The conformation of the isolated FADD-DED was determined by circular dichroism (CD) analysis (JASCO 715). FADD-DED generated by subtilisin digest was isolated as described above and dissolved at 0.1 mg/ml in 10 mM sodium phosphate (pH 7.0) and CD spectrum was generated by scanning from 190 to 260 nm at 25°C.

### (Example 2) Construction of FADD Mutants

The DNA fragments encoding proteolytically determined DED (Met¹-Thr⁸⁸) and DD (Ala⁸⁹-Ser¹⁸³) of FADD were isolated by PCR using their specific primers and cloned into pET3d and pET14b, respectively, using *Nco*I and *Bam*HI sites that were artificially designed into the primers. The Asp¹⁹Ala, Phe²⁵Ala and Asp⁷⁴Ala mutants were generated PCR mutagenesis using the specific mutagenic PCR primers. The primers for the 5' and 3' ends of the gene for FADD-DED were paired with each of the mutagenic primers. The two PCR fragments containing the same region of the mutated site were obtained and mixed again for the second PCR. The two fragments were then combined during the second PCR to cover the whole structural region for FADD-DED. The mutated genes for FADD-DED were then cleaved with *Nco*I and *Bam*HI and cloned into the same sites of pET3d. The mutations were confirmed by DNA sequencing.

### (Example 3) Determination of Cell Viability and Morphology

The DNA fragment encoding FADD-DED was cleaved with *Bgl*II and *Hind*III and transferred into the same sites of pET28a. Since pET28a contains *lac*I gene, the expression of FADD-DED is more tightly repressed in the absence of IPTG. *E. coli* BL21(DE3) cells harboring pET28a-FADD-DED were grown in LB broth containing ampicillin at 50 µg/ml. Expression of FADD-DED was induced by adding different concentrations of IPTG and the cell growth was measured by OD₆₀₀. To determine the number of the viable cells, the culture broth was sampled at time interval after 0.5 mM IPTG induction of FADD-DED and the sampled cells were plated on LB plate containing ampicillin. To monitor cell morphology, the sampled cells were stained with 1 % crystal violet and observed by light microscopy at 1,200 magnitude. The effect of FADD-DED on the mammalian cell was tested using HeLa cells. The structural gene for FADD-DED was obtained by PCR using the specific primers. The PCR product was cleaved with *Eco*RI and *Sal*I that were designed into the primers and cloned into the mammalian expression vector, pcDNA3 (Invitrogen) cleaved with *Eco*RI and *Xho*I. The resulting plasmid was then transfected into HeLa cells using GenePorter (Gene Therapy System). The β-galactosidase gene cloned in the same vector was co-transfected to identify the transfected cells. The cells were fixed with 0.2% glutaraldehyde and 2% formaldehyde in 24 hours after transfection, and then stained with 5-bromo-4-chloro-3-indolyl β -D-galactoside for 3~5 hours. The apoptotic cells were determined by apoptotic morphology among the transfected cells (Yang *et al*., 1997). At least 500 β-galactosidase positive cells were scored for each transfection in triplicate and the mean percentages of apoptotic blue cells and their standard errors were determined.

### (Example 4) Analysis of Nicked DNA

The DNA nicking induced by FADD-DED was analyzed as previously described (Asoh *et al*., 1998). E. *coli* BL21(DE3) cells containing pET28a-FADD-DED or pET28a vector were cultured in LB broth at 37°C until OD₆₀₀ reached 0.8. Expression of FADD-DED was induced by adding 0.5 mM IPTG. The cells were harvested at different time interval and lyzed with 1N NaOH. Chromosomal DNA was alkaline denatured and the nicked DNA was separated from intact DNA by ultracentrifugation at 100,000 g for 1 hour. The single stranded DNA in the supernatant was precipitated with ethanol and then dissolved in TE buffer (10 mM Tris-HCl, pH 8.0, 1 mM EDTA). The contaminating RNA was removed by RNase A treatment and the remained DNA was precipitated with isopropanol again. The pellet was redissolved in TE buffer and resolved in a 1% alkaline agarose gel (50 mM NaOH, 1 mM EDTA). The single stranded DNA was then transferred onto a nylon membrane (Hybond-N, Amersham Pharmacia Biotech). The transferred DNAs on the membrane were hybridized with digoxigenin-labeled probe prepared by PCR with random primer using *E*. *coli* genomic DNA as a template.

### (Example 5) Determination of Mutation Frequency

To see whether FADD-DED expression increases mutation, the frequency of rifampicin-resistant cells was determined as previously described with a slight modification (Asoh *et al*., 1998). *E. coli* BL21(DE3) cells containing either pET28a-FADD-DED or the vector alone were incubated on LB plate containing kanamycin for 16 hours. The colonies on each plate were pooled and the 1 x 10¹⁰ cells were spread on kanamycin-containing LB plate with and without rifampicin (50 µM) that was chosen as an indicative marker to monitor spontaneous mutation frequency. The colonies on each plate were counted to determine the frequency of the rifampicin-resistant cells.

### (Example 6) Immunoblotting of FADD-DED

*E. coli* BL21(DE3) cells expressing FADD-DED were lyzed and the proteins were separated by SDS-polyacrylamide gel electrophoresis. The proteins in the gel were transferred to PVDF membrane (Millipore) and FADD-DED was bound by immunoblotting with anti-human FADD mouse antibody (Pharmingen). The antibody complex was then detected by enhanced chemiluminescence system (Amersham Pharmacia Biotech).

### (Example 7) Co-expression of Thioredoxin or SOD with FADD-DED

The thioredoxin gene of *E*. *coli* was isolated by PCR with the specific primers and cloned at *Bam*HI site of pACYC184 to generate pETX (K.-S. Kim, unpublished). This recombinant plasmid was transformed into *E. coli* BL21(DE3) and AD494(DE3) (*Δara-leu7967, Δ lacX74, ΔphoApvuII phoR ΔmalF3,* F'[*lac*^{*+*}*(lacIq)pro*]*trxB::kan*(DE3)) which is defective mutant of thioredoxin reductase derived from BL21(DE3). The transformants were selected on LB plates containing chloramphenicol. To each of the transformed cells, pET28a-FADD-DED was introduced and the transformants were selected on LB plates containing chloramphenicol and ampicillin. Expression of FADD-DED was then induced on LB plate containing 0.5 mM IPTG. To compare the killing effect of FADD-DED with other toxic peptide, another plasmid, pGATA (purchased from CPC Inc. USA), expressing a toxic peptide derived from mouse transcription factor GATA-1 was also transformed into the same tester strains. The gene encoding Fe-superoxide dismutase (SOD) of *Aquifex pyrophilus* was cloned at *NcoI* and *Bam*HI sites of pET3c to generate pSOD (Lim et al., 1997). The FADD-DED gene cloned at *Bgl*II/*Hind*III was cut from pET3d and cloned into pACYC184. Expression of FADD-DED from pACYC184-FADD-DED was also induced by IPTG in *E. coli*.

### (Example 8) Cell Growth in the Presence of Vitamin E or in Anaerobic Condition

The *E. coli* BL21(DE3) containing pET28a-FADD-DED was cultured in LB broth containing ampicillin and 0.5 mM IPTG was added to induce FADD-DED. Vitamin E was added 1 hour after IPTG induction and cell growth was monitored by OD₆₀₀. The same cells were distributed into 1.5 ml Eppendorf tubes completely filled with LB broth and cultivated without agitation until OD₆₀₀ reached 0.5. Then, after IPTG was added at 0.5 mM, the cell growth was monitored.

### (Example 9) Genetic Suppression of FADD-DED with E. coli genes

The *E. coli* chromosome was digested with *Bam*HI and the 3-5 kb genomic fragments were ligated into the *Bam*HI site of pBR322. The ligation mixture was transformed into *E. coli* DH5a and the transformants were pooled to isolate the plasmids. The plasmids were retransformed into E. *coli* BL21(DE3). Then, pACYC184-FADD-DED was additionally transformed into the cells containing pBR322 containing each of the *E*. *coli* genes. The transformants forming colonies were selected on LB plates containing ampicillin and chloramphenicol (each 30 µg/ml). The plasmids were isolated from the viable cells to determine the *E*. *coli* genes cloned in pBR322.

### (REFERENCES)

1. Ameisen, J.C. (1996) The origin of programmed cell death. S*cience* **272:** 1278-1279
2. Ames, B. N., and Gold, L. S. (1991) Endogenous mutagens and the causes of aging and cancer. *Mutat Res* **250:** 3-16
3. Aravind, L., Dixit, V. M., and Koonin, E.V. (1999) The domains of death: evolution of the apoptosis machinery. *Trends Biochem Sci* **24:** 47-53
4. Asoh, S., Nishimaki, K., Nanbu-Wakao, R., and Ohta, S. (1998) A trace amount of the human pro-apoptotic factor Bax induces bacterial death accompanied by damage of DNA. *J Biol Chem* **273:** 11384-11391
5. Brandi, G^{a}., Fiorani, M., Pierotti, C., Albano, A., Cattabeni, F., and Cantoni, O. (1989) Morphological changes in *Escherichia coli* cells exposed to low or high concentrations of hydrogen peroxide. *Microbiol Immunol* **33:** 991-1000
6. Brandi, G^{b}., Schiavano, G. F., Albano, A., Cattabeni, F., and Cantoni, O. (1989) The effect of K₂CrO₇ on the growth and morphology of *Escherichia coli. Biol Trac Elem Res* **21:** 271-275
7. Chinnaiyan, A. M., O'Rourke, K., Tewari, M., and Dixit,V. M. (1995) FADD, a novel death domain-containing protein, interacts with the death domain of Fas and initiates apoptosis. *Cell* **81:** 505-512
8. Chinnaiyan, A. M., Tepper, C. G., Seldin, M. F., O'Rourke, K., Kischkel, F. C., Hellbardt, S., Krammer, P. H., Peter, M. E., and Dixit, V. M. (1996) FADD/MORT1 is a common mediator of CD95 (Fas/APO-1) and tumor necrosis factor receptor-induced apoptosis. *J Biol Chem* **271:** 4961-4965
9. Cohen, G. M. (1997) Caspases: the executioners of apoptosis. *Biochem J* **326:** 1-16
10. Cornillon, S., Foa, C., Davoust, J., Buonavista, N., Gross, J. D., and Golstein, P. (1994) Programmed cell death in *Dictyostelium. J Cell Sci* **107:** 2691-2704
11. Das, K. C., Lewis-Molock, Y., and White, C. W. (1997) Elevation of manganese superoxide dismutase gene expression by thioredoxin. *Am J Respir Cell Mol Biol* **17:** 713-726
12. Eberstadt, M., Huang, B., Chen, Z., Meadows, R. P., Ng, S. C., Zheng, L., Lenardo, M. J., and Fesik, S. W. (1998) NMR structure and mutagenesis of the FADD (Mort1) death-effector domain. *Nature* **392:** 941-945
13. Felzenszwalb, I., da Silva, S. R., and Alcantara Gomes, R. (1987) Effect of hydrogen peroxidase on *Escherichia coli radC. Braz J Med Biol Res* **20:** 869-871
14. Fernando, M. R., Nanri, H., Yoshitake, S., Nagata-Kuno, K., Minakami, S. (1992) Thioredoxin regenerates proteins inactivated by oxidative stress in endothelial cells. *Eur J Biochem* **209:** 917-922
15. Fuentes, A. M., and Amabile-Cuevas, C. F. (1998) Antioxidant vitamins C and E affect the superoxide-mediated induction of the soxRS regulon of *Escherichia coli. Microbiology* **144:** 1731-1736
16. Gyuris, J., Golemis, E., Chertkov, H., and Brent, R. (1993) Cdi1, a human G1 and S phase protein phosphatase that associates with Cdk2. *Cell* **75:** 791-803
17. Hochman, A. (1997) Programmed cell death in prokaryotes. *Crit Rev Microbiol* **23:** 207-214
18. Hrebenda, L., Hrebenda, B., and Brozostek, K. (1985) Influence of penicillin and nalidixic acid on growth and cell division of *Escherichia coli* K-12. *Acta Microbiol Pol* **34:** 19-24
19. Huang, B., Eberstadt, M., Olejniczak, E. T., Meadows, R. P., and Fesik, S. W. (1996) NMR structure and mutagenesis of the FAS (APO-1/CD95) death domain. *Nature* **384:** 638-641
20. Ishibashi, Y., Nishimaki, K., Asoh, S., Nanbu-Wakao, R., Yamada, T., and Ohta, S. (1998) Pore formation domain of human pro-apoptotic bax induces mammalian apoptosis well as bacterial death without antagonizing anti-apoptotic factors. *Biochem Biophys Res Commun* **243:** 609-616
21. Jeong, E. J., Bang, S., Lee., T. H., Park, Y. I., Sim, W. S., and Kim, K. S. (1999) The solution structure of FADD death domain. Structural basis of death domain interactions of Fas and Fadd. *J Biol Chem* **274:** 16337-16342
22. Juo, P., Kuo, C. J., Yuan, J., and Blenis, J. (1998) Essential requirement for caspase-8/FLICE in the initiation of the Fas-induced apoptotic cascade. *Curr Biol* **8:** 1001-1008.
23. Keyer, K., Gort, A. S., and Imlay, J. A. (1995) Superoxide and the production of oxidative DNA damage. *J Bacteriol* **177:** 6782-6790
24. Lennon, B. W., and Williams, C. H. Jr. (1996) Enzyme-monitored turnover of Escherichia coli thioredoxin reductase: insights for catalysis. *Biochemistry* **35:** 4704-4712
25. Li, H., Zhu, H., Xu, C. J., and Yuan, J. (1998) Cleavage of BID by caspase 8 mediates the mitochondrial damage in the Fas pathway of apoptosis. *Cell* **94:** 491-501
26. Lim, J. H., Yu, Y. G., Choi, I. G., Ryu, J. R., Ahn, B. Y., Kim, S. H., and Han, Y. S. (1997) Cloning and expression of superoxide dismutase from Aquifex pyrophilus, a hyperthermophilic bacterium. *FEBS Lett* **406:** 142-146
27. Medema, J. P., Scaffidi, C., Kischkel, F. C., Shevchenko, A., Mann, M., Krammer, P. H., and Peter, M. E. (1997) FLICE is activated by association with the CD95 death-inducing signaling complex (DISC). *EMBO J* **16:** 2794-2804
28. Moody, C. S., and Hassan, H. M. (1982) Mutagenicity of oxygen free radicals. *Proc Natl Acad Sci USA* **79:** 2855-2859
29. Muzio, M., Chinnaiyan, A. M., Kischkel, F. C., O'Rourke, K., Shevchenko, A., Ni. J., Scaffidi, C., Bretz, J. D., Zhang, M., Gentz, R., Mann, M., Krammer, P. H., Peter, M. E., and Dixit, V. M. (1996) FLICE, a novel FADD-homologous ICE/CED-3-like protease, is recruited to the CD95 (Fas/APO-1) death-inducing signaling complex. *Cell* **85:** 817-827
30. Naito, T., Kusano, K., and Kobayashi, I. (1995) Selfish behavior of restriction-modification systems. *Science* **267:** 897-899
31. Phillips, S. L., Person, S., and Jagger, J. (1967) Division delay induced in *Escherichia coli* by near-ultraviolet *radiation. J Bacteriol* **94:** 165-170
32. Prinz, W. A., Aslund, F., Holmgren, A., and Beckwith, J. (1997) The role of the thioredoxin and glutaredoxin pathways in reducing protein disulfide bonds in the *Escherichia coli* cytoplasm. *J Biol Chem* **272:** 15661-15667
33. Spector, A., Yan, G. Z., Huang, R. R., McDermott, M. J., Gascoyne, P. R., and Pigiet, V. (1988) The effect of H₂O₂ upon thioredoxin-enriched lens epithelial cells. *J Biol Chem* **263:** 4984-4990
34. Steller, H. (1995) Mechanisms and genes of cellular suicide. *Science* **267:** 1445-1449
35. Storz, G., and Imlay, J. A. (1999) Oxidative stress. *Curr Opinion Microbiol* **2:** 188-194
36. Suzuki, H., Pangborn, J., and Kilgore, W. W. (1967) Filamentous cells of *Eschcrichia coli* formed in the presence of mitomycin. *J Bacteriol **93:*** 683-688
37. Suzuki, Y., Ono, Y., and Hirabayashi, Y. (1998) Rapid and specific reactive oxygen species generation via NADPH oxidase activation during Fas-mediated apoptosis. *FEBS Lett* **425:** 209-212
38. Takemoto, T., Zhang, Q. M., and Yonei, S. (1998) Different mechanisms of thioredoxin in its reduced and oxidized forms in defense against hydrogen peroxide in *Escherichia coli. Free Radic Biol Med* **24:** 556-562
39. Trudel, P., Provost, S., Massie, B., Chartrand, P., and Wall, L. (1996) pGATA: a positive selection vector based on the toxicity of the transcription vector GATA-1 to bacteria. *BioTechniques* **20:** 684-693
40. Welbum, S. C., Lillico, S., and Murphy, N. B. (1999) Programmed cell death in procyclic form *Trypanosoma brucei rhodesiense* - Identification of differentially expressed genes during con A induced death. *Mem Inst Oswaldo Cruz* **94:** 229-234
41. Wheatley, D. N., Christensen, S. T., Schousboe, P., and Rasmussen, L. (1993) Signaling in cell growth and death: adequate nutrition alone may not be sufficient for ciliates. *Cell Biol Int* **17:** 817-823
42. Xiang, J., Chao, D. T., and Korsmeyer, S. J. (1996) BAX-induced cell death may not require interleukin 1 beta-converting enzyme-like proteases. *Proc Natl Acad Sci USA* **93:** 14559-14563
43. Yang, X., Khosravi-Far, R., Chang, H. Y., and Baltimore, D. (1997) Daxx, a novel Fas-binding protein that activates JNK and apoptosis. *Cell* **89**: 1067-1076

## Claims

1. A death effector domain, FADD-DED consisting of SEQ ID NO: 3 of mammalian apoptosis mediator, wherein FADD-DED induces bacterial cell death.

2. A kit for screening the molecules related to apoptosis, comprising an expression vector comprising a polynucleotide encoding exclusively the FADD-DED of claim 1 and T7 promoter.

3. The kit according to claim 2, further comprising E. coli in which T7 polymerase is not present and E. coli in which T7 polymerase is expressed by TPTG induction.

4. A polynucleotide encoding exclusively the FADD-DED of claim 1.

5. A vector comprising the polynucleotide of claim 4.

6. A host cell comprising the vector of claim 5.

## Patentansprüche

1. Todeseffektordomäne FADD-DED, bestehend aus SEQ ID NO: 3 eines Säuger-Apoptose-Vermittlers, wobei FADD-DED bakteriellen Zelltod induziert.

2. Kit zum Screenen der Moleküle, die mit Apoptose zusammenhängen, umfassend einen Expressionsvektor, der ein Polynucleotid umfasst, das ausschließlich das FADD-DED nach Anspruch 1 und einen T7-Promotor codiert.

3. Kit nach Anspruch 2, das zusätzlich *E.coli* umfasst, in welchen die T7-Polymerase nicht vorhanden ist, und *E. coli,* in welchen die T7-Polymerase durch IPTG-Induktion exprimiert wird.

4. Polynucleotid, das ausschließlich das FADD-DED nach Anspruch 1 codiert.

5. Vektor, umfassend das Polynucleotid nach Anspruch 4.

6. Wirtszelle, umfassend den Vektor nach Anspruch 5.

## Revendications

1. Domaine effecteur de mort, FADD-DED, consistant en SEQ ID NO : 3 du médiateur d'apoptose mammifère, dans lequel FADD-DED induit la mort de cellules bactériennes.

2. Trousse de criblage de molécules apparentées à l'apoptose, comprenant un vecteur d'expression comprenant un polynucléotide codant exclusivement le domaine FADD-DED de la revendication 1 et le promoteur T7.

3. Trousse selon la revendication 2, comprenant en outre E. coli dans laquelle la polymérase T7 n'est pas présente et E. coli dans laquelle la polymérase T7 est exprimée par induction par IPTG.

4. Polynucléotide codant exclusivement le FADD-DED selon la revendication 1.

5. Vecteur comprenant le polynucléotide selon la revendication 4.

6. Cellule hôte comprenant le vecteur selon la revendication 5.


### SEQUENCE LISTING

<110> Imagene Co., Ltd.
<120> Death effector domain of mammalian apoptosis mediator, FADD, inducing bacterial cell death
<130> MK-95/IM/PCT
<150> KR 99-27964
   <151> 1999-07-12
<160> 4
<170> KOPATIN 1.5
<210> 1
   <211> 1377
   <212> DNA
   <213> mouse
<400> 1
   cgagatttac ccatggaccc attcctggtg ctgctgcact cgctgtccgg cagcctgtcg      60
   ggcaacgatc tgatggagct caagttcttg tgccgcgagc gcgtgagcaa acgaaagctg     120
   gagcgcgtgc agagtggcct ggacctgttc acggtgctgc tggagcagaa cgacctggag     180
   cgcgggcaca ccgggctgct gcgcgagttg ctggcctcgc tgcgccgaca cgatctactg     240
   cagcgcctgg acgacttcga ggcggggacg gcgaccgctg cgcccccggg ggaggcagat     300
   ctgcaggtgg catttgacat tgtgtgtgac aatgtgggga gagactggaa aagactggcc     360
   cgcgagctga aggtgtctga ggccaagatg gatgggattg aggagaagta cccccgaagt     420
   ctgagtgagc gggtaaggga gagtctgaaa gtctggaaga atgctgagaa gaagaacgcc     480
   tcggtggccg gactggtcaa ggcgctgcgg acctgcaggc tgaatctggt ggctgacctg     540
   gtggaagaag cccaggaatc tgtgagcaag agtgagaata tgtccccagt actaagggat     600
   tcaactgtgt cttcctcaga aacaccctga caagcctaca gctcctctca ggagtcactg 660
   tggacttgat cacaccatga tgtagcacca agtgcctttt gtgcccagtg ccaaaaagat 720
   cttgctgatc cagggcgggt cccctcccac cagctcctgg agtatatctg tatctacgga 780
   acctctgctc cctgggcttt cggaacacat gaaggttccc cgtcttgttg tccaccactg 840
   cccccccccc ccagaggtct ctggcttgca agaaggaagt tggagctgat ggcacatgtg 900
   ctactgcaat ttgagagcct tcttcttgtt ccttggggga agacaccatc tcagttaagt 960
   cacttggttg ccttacagtt ggccaagagg acatctgtct cacatccttg gaggccatca 1020
   gacagcagct cctgaccccc caccctcaaa caaggtgcaa gacccctgtg aggactgtgg 1080
   tttaaagggt agcaccttgc ctccatggtg gtctgtaaga agtgcctggc ctacctacat 1140
   atagtggctc actctgaaat cctagcactt gagagttgag aactgcactt tacagcagcc 1200
   tgagctacaa atgagatgtt ctccccaaaa cacacaaaag caagtgcctt tacctggaga 1260
   taacttgtgt caccaagaat ttctattata ttaagaaccc ttctaacctc agaagtccct 1320
   cccatcaggc ttgactaagg tgtgcttaca gccctgggtg ctagttgtga ggtaaat 1377
<210> 2
   <211> 205
   <212> PRT
   <213> mouse
<400> 2
   Met Asp Pro Phe Leu Val Leu Leu His Ser Leu Ser Gly Ser Leu Ser
   1 5 10 15
   Gly Asn Asp Leu Met Glu Leu Lys Phe Leu Cys Arg Glu Arg Val Ser
   20 25 30
   Lys Arg Lys Leu Glu Arg Val Gln Ser Gly Leu Asp Leu Phe Thr Val
   35 40 45
   Leu Leu Glu Gln Asn Asp Leu Glu Arg Gly His Thr Gly Leu Leu Arg
   50 55 60
   Glu Leu Leu Ala Ser Leu Arg Arg His Asp Leu Leu Gln Arg Leu Asp
   65 70 75 80
   Asp Phe Glu Ala Gly Thr Ala Thr Ala Ala Pro Pro Gly Glu Ala Asp
   85 90 95
   Leu Gln Val Ala Phe Asp Ile Val Cys Asp Asn Val Gly Arg Asp Trp
   100 105 110
   Lys Arg Leu Ala Arg Glu Leu Lys Val Ser Glu Ala Lys Met Asp Gly
   115 120 125
   Ile Glu Glu Lys Tyr Pro Arg Ser Leu Ser Glu Arg Val Arg Glu Ser
   130 135 140
   Leu Lys Val Trp Lys Asn Ala Glu Lys Lys Asn Ala Ser Val Ala Gly
   145 150 155 160
   Leu Val Lys Ala Leu Arg Thr Cys Arg Leu Asn Leu Val Ala Asp Leu
   165 170 175
   Val Glu Glu Ala Gln Glu Ser Val Ser Lys Ser Glu Asn Met Ser Pro
   180 185 190
   Val Leu Arg Asp Ser Thr Val Ser Ser Ser Glu Thr Pro
   195 200 205
<210> 3
   <211> 88
   <212> PRT
   <213> mouse
<400> 3
   Met Asp Pro Phe Leu Val Leu Leu His Ser Leu Ser Gly Ser Leu Ser
   1 5 10 15
   Gly Asn Asp Leu Met Glu Leu Lys Phe Leu Cys Arg Glu Arg Val Ser
   20 25 30
   Lys Arg Lys Leu Glu Arg Val Gln Ser Gly Leu Asp Leu Phe Thr Val
   35 40 45
   Leu Leu Glu Gln Asn Asp Leu Glu Arg Gly His Thr Gly Leu Leu Arg
   50 55 60
   Glu Leu Leu Ala Ser Leu Arg Arg His Asp Leu Leu Gln Arg Leu Asp
   65 70 75 80
   Asp Phe Glu Ala Gly Thr Ala Thr
   85
<210> 4
   <211> 117
   <212> PRT
   <213> mouse
<400> 4
   Ala Ala Pro Pro Gly Glu Ala Asp Leu Gln Val Ala Phe Asp Ile Val
   1 5 10 15
   Cys Asp Asn Val Gly Arg Asp Trp Lys Arg Leu Ala Arg Glu Leu Lys
   20 25 30
   Val Ser Glu Ala Lys Met Asp Gly Ile Glu Glu Lys Tyr Pro Arg Ser
   35 40 45
   Leu Ser Glu Arg Val Arg Glu Ser Leu Lys Val Trp Lys Asn Ala Glu
   50 55 60
   Lys Lys Asn Ala Ser Val Ala Gly Leu Val Lys Ala Leu Arg Thr Cys
   65 70 75 80
   Arg Leu Asn Leu Val Ala Asp Leu Val Glu Glu Ala Gln Glu Ser Val
   85 90 95
   Ser Lys Ser Glu Asn Met Ser Pro Val Leu Arg Asp Ser Thr Val Ser
   100 105 110
   Ser Ser Glu Thr Pro
   115